# EUROPÄISCHE PATENTSCHRIFT

(11) **EP 1 107 799 B1**
(45) Veröffentlichungstag und Bekanntmachung des Hinweises auf die Patenterteilung: **04.02.2004**
(21) Anmeldenummer: 99946009.0
(22) Anmeldetag: 21.08.1999
(51) Int. Cl.: A61K 51/12, A61K 49/00

(54) **VERFAHREN ZUR MARKIERUNG VON BIOPOLYMEREN MIT ISOTOPEN**
METHOD FOR LABELING BIOPOLYMERS USING ISOTOPES
TECHNIQUE DE MARQUAGE ISOTOPIQUE DE BIOPOLYMERES

(30) Priorität: 29.08.1998 DE 19839491
(43) Veröffentlichungstag der Anmeldung: 20.06.2001
(73) Patentinhaber: Silantes GmbH, 80339 München (DE)
(72) Erfinder: HEUMANN, Hermann, D-82166 Gräfelfing (DE)
(74) Vertreter: Maiwald, Walter, Dr. Dipl.-Chem.
(86) Internationale Anmeldenummer: PCT/EP1999/006154
(87) Internationale Veröffentlichungsnummer: WO 2000/012140

(56) Entgegenhaltungen:
- D.G. DONNE ET AL.: "Structure of the recombinant full-lenght hamster prion protein PrP (29-231): The N terminus is highly flexible" PROCEEDINGS OF THE NATIONAL ACADEMY OF SCIENCES OF USA, Bd. 94, 1997, Seiten 13452-13457, XP002128049 WASHINGTON US in der Anmeldung erwähnt
- G. MER ET AL. : "Enzymatic synthesis of region-specific isotope-labeled DNA oligomers for NMR analysis" JOURNAL OF THE AMERICAN CHEMICAL SOCIETY, Bd. 120, 1998, Seiten 607-608, XP002128050 DC US in der Anmeldung erwähnt
- BATEY, ROBERT T. ET AL: "Preparation of isotopically labeled ribonucleotides for multidimensional NMR spectroscopy of RNA" NUCLEIC ACIDS RES. (1992), 20(17), 4515-23 , XP002128051
- DATABASE CHEMABS [Online] CHEMICAL ABSTRACTS SERVICE, COLUMBUS, OHIO, US STEGMANN, RENATA ET AL: "Structural changes of the Escherichia coli GroEL - GroES chaperonins upon complex formation in solution: a neutron small angle scattering study" retrieved from STN Database accession no. 129:65069 XP002128053 & J. STRUCT. BIOL. (1998), 121(1), 30-40 ,
- BATEY, ROBERT T. ET AL: "Improved large scale culture of Methylophilus methylotrophus fo 13C/15N labeling and random fractional deuteration of ribonucleotides" NUCLEIC ACIDS RES. (1996), 24(23), 4836-4837 , XP002128052
- DATABASE MEDLINE [Online] US NATIONAL LIBRARY OF MEDICINE (NLM), BETHESDA, MD, US BATEY R T ET AL: "Preparation of isotopically enriched RNAs for heteronuclear NMR." retrieved from STN Database accession no. 96139821 XP002128054 in der Anmeldung erwähnt & METHODS IN ENZYMOLOGY, (1995) 261 300-22. ,

## Beschreibung

Die vorliegende Erfindung betrifft ein neues Verfahren zur in vivo-Markierung von Biopolymeren, wie Proteinen, Nukleinsäuren, Lipiden, Kohlenhydraten und biodegradierbarem Kunststoff, mit Isotopen, insbesondere stabilen Isotopen, und die Verwendung von chemolithotrophen Bakterien zur *in vivo*-Isotopenmarkierung von Biopolymeren. Insbesondere betrifft die Erfindung den Einsatz von CO₂-fixierenden Bakterien, wie *Ralstonia eutropha* und ähnlichen Knallgasbakterien, zur *in vivo*-Isotopenmarkierung, insbesondere zur Markierung mit dem stabilen Isotop ¹³C, und die Verwendung der isotopenmarkierten Biomoleküle in therapeutischen und diagnostischen Anwendungen, insbesondere in Spektroskopieverfahren und allgemein als Tracerverbindungen.

Der gezielte Einsatz von Biomolekülen in Diagnostik und Therapie erfordert Kenntnisse über die Struktur und Dynamik dieser Moleküle. Diese Informationen können beispielsweise durch den Einsatz der Kernspinresonanz (Nuclear Magnetic Resonance, NMR)-Technik erhalten werden. Voraussetzung für einen effizienten Einsatz dieser Methode ist allerdings die Markierung der interessierenden Biomoleküle mit stabilen Isotopen, sog. S-Isotopen, wie beispielsweise ²H, ¹³C und ¹⁵N.

Da S-Isotope nicht radioaktiv und mit Ausnahme von Deuterium auch nicht toxisch sind, haben sie ein großes diagnostisches Potential. Insbesondere in der Stoffwechseldiagnostik werden S-Isotope bereits erfolgreich eingesetzt. Neben ²H, ¹³C und ¹⁵N bieten sich vor allem die Kerne ¹H, ⁷Li, ¹¹B, ¹⁴N, ¹⁷O, ¹⁸O, ¹⁹F, ²³Na, ²⁹Si, ³¹P, ³³S und ⁷⁷Se für die NMR-Spektroskopie organischer Verbindungen an (vgl. z.B. Vogel H.J. (1989) Methods in Enzymology Vol. 177, 263).

In der bildgebenden NMR-Spektroskopie, speziell in der NMR-Mikroskopie, wird der Einsatz von S-isotopenmarkierten Substanzen, insbesondere von markierten Aminosäuren, diskutiert. Mit der Weiterentwicklung von bildgebenden NMR-Verfahren wird sich der Einsatzbereich von S-isotopenmarkierten Substanzen erweitern und nicht nur auf die oben genannten S-Isotope ²H, ¹³C und ¹⁵N beschränkt bleiben.

Bislang erfolgt die vollständige S-Isotopenmarkierung von Proteinen und anderen Biopolymeren im allgemeinen durch *in vivo*-Markierung, d.h. Organismen werden auf isotopenmarkierten Medien gezogen und aus den markierten Organismen wird anschließend das gewünschte Protein oder eine andere Komponente isoliert.

Das für die Aufzucht notwendige Medium, das in der Regel aus Kohlenstoffquelle, Stickstoffquelle und Salzen besteht, ist um so aufwendiger und kostenintensiver, je komplexer es ist. Dies gilt insbesondere für die Kohlenstoffquelle. Da Nährmedien für höhere, also eukaryontische Organismen besonders komplex sind, erfolgt die Expression von S-isotopenmarkierten eukaryontischen Proteinen meist rekombinant in Bakterien, und zwar in den meisten Fällen in *Escherichia coli* (siehe z.B. Donne D.G. *et al*. (1997) Proc. Natl. Acad. Sci. USA 94, 13452-13457). Dieses Bakterium verwendet als preisgünstigste ¹³C-markierte Kohlenstoffquelle Glucose. Alternativ wurde auch ¹³C-markiertes Methanol, das relativ preisgünstig ist, für die Zucht von *Methylophilus methylotrophus* eingesetzt (Batey R. *et al*. (1995) Methods in Enzymology 261, 300-322). Die kostengünstigste Kohlenstoffquelle ist ¹³Cmarkiertes CO₂. Grünalgen (wie z.B. *Chlorella vulgaris, Chlorella pyrenoidosa, Chlorella fusca* oder *Scenedesmus obliquus*) können Kohlendioxid mittels photosynthetischer Reduktion fixieren und somit durch Verfütterung von ¹³Cmarkiertem CO₂ vollständig markiert werden. Das markierte Algenhydrolysat wird dann wiederum als Kohlenstoffquelle für die Zucht von Bakterien, insbesondere *E. coli*, eingesetzt.

Die Verwendung von Algenhydrolysat statt Glucose ist nicht nur wirtschaftlicher, sondern in vielen Fällen aus biologischen Gründen auch notwendig. Werden in *E*. *coli* fremde Proteine exprimiert, sinkt oft die erreichbare Zelldichte, insbesondere wenn Minimalmedium mit Glucose als Kohlenstoffquelle verwendet wird. In vielen Fällen wird die Expression eines heterologen Proteins in *E. coli* erst durch den Einsatz von Algenhydrolysat als C-Quelle möglich; häufig kann zumindest die Ausbeute an heterolog exprimiertem Protein und damit die Wirtschaftlichkeit des Markierungsverfahrens durch die Verfütterung von Algenhydrolysat gesteigert werden.

Für die Herstellung von S-isotopenmarkierten Nukleinsäuren, insbesondere DNA, wird meist Plasmid-DNA aus *E. coli* derart manipuliert, daß die gewünschte Nukleinsäuresequenz auf dem Plasmid dargestellt wird. Eine alternative Methode, die für RNA obligatorisch und für DNA optional ist, ist die Isolierung der gesamten RNA und DNA aus markierten Zellen (*E. coli* oder Algen). Nach Hydrolyse der DNA bzw. RNA, Isolierung der Nukleotide und Phosphorylierung zu Ribonukleosidtriphosphaten bzw. Deoxyribonukleosidtriophosphaten werden neue Nukleinsäuren mit geeigneten Polymerasen *in vitro* synthetisiert (Mer G. and Chazin W.J. (1998) J. Am. Chem. Soc. 120, 607-608).

Im Stand der Technik erfolgt die Markierung mit ¹³C somit mittels komplexer - und damit aufwendiger - Kohlenstoffquellen wie ¹³C-Glucose im Falle *der in vivo*-Markierung von Bakterien, wie *E. coli*, oder alternativ unter Einsatz von ¹³Cmarkiertem Kohlendioxid im Falle der *in vivo*-Markierung von Grünalgen und anschließender Verfütterung des ¹³C-markierten Algenhydrolysats als C-Quelle.

Die Markierung mit ¹⁵N erfolgt in der Regel durch Verwendung von entsprechend markierten Salzen, die Markierung mit ²H durch Anzucht in D₂O. Die Markierung mit anderen Isotopen erfolgt analog durch Verstoffwechselung entsprechend markierter Substanzen.

In vielen Fällen ist eine Markierung mit mehreren S-Isotopen notwendig. Eine Doppel- oder Mehrfach-Markierung kann durch Kombination der verschiedenen Methoden erreicht werden.

Auch radioaktiv markierte Biopolymere finden vielfältigen Einsatz in Therapie und Diagnostik. So werden mit radioaktiven Isotopen markierte Biomoleküle z.B. als Tracer oder Marker bei der Prüfung von Stoffwechsel- und Kreislauffunktionen, bei der Verfolgung und Sichtbarmachung von Anreicherungsprozessen in Geweben und Organen, bei radioimmunologischen und verwandten *in vitro*-Meßmethoden, als Radiopharmaka in der nuklearmedizinischen *in vivo*-Diagnostik (z.B. Szintigraphie) und in der Autoradiographie eingesetzt.

Eine Aufgabe der Erfindung besteht darin, ein neues Verfahren zur *in vivo*-Markierung von Biomolekülen mit Isotopen bereitzustellen.

Eine besondere Aufgabe besteht darin, ein Verfahren zur *in vivo*-Markierung von Biopolymeren mit stabilen Isotopen, insbesondere mit ¹³C, allein und in Kombination mit anderen Isotopen bereitzustellen.

Eine weitere Aufgabe besteht darin, ein *in vivo*-Markierungsverfahren zur Verfügung zu stellen, das die Nachteile der Verfahren des Standes der Technik überwindet und im Vergleich zu bekannten Verfahren effizienter und kostengünstiger durchgeführt werden kann.

Diese und weitere Aufgaben werden durch das erfindungsgemäße Verfahren gelöst, das auf dem Einsatz chemolithotropher Bakterien basiert und in den unabhängigen Ansprüchen definiert ist. Bevorzugte Ausführungsformen der Erfindung ergeben sich aus den Unteransprüchen.

In einer besonderen Ausführungsform des Verfahrens erfolgt die *in vivo*-Markierung von Biopolymeren mit Isotopen, bevorzugt stabilen Isotopen, mittels CO₂-fixierender Bakterien, wobei in einer bevorzugten Ausführungsform die Markierung mit stabilen und/oder instabilen Kohlenstoffisotopen in Knallgasbakterien stattfindet. Beispiele für Knallgasbakterien, die für das erfindungsgemäße *in vivo*-Markierungsverfahren geeignet sind, sind *Acidovorax facilis, Alcaligenes ruhlandii, Alcaligenes latus, Alcaligenes* sp. 2625, *Ancylobacter aquaticus, Ancylobacter* sp. 1106-1108, 2456, 2457, 2666-2669, *Aquifex pyrophilus, Aquaspirillum autotrophicum, Azospirillum amazonense, Azospirillum* sp 1726, 1727, *Azospirillum lipoferum, Azotobacter* sp. 1721-1723, *Bacillus* schlegelii, *Bradyrhizobium japnonicum, Bacillus tusciae, Calderobacterium hydrogenophilum, Campylobacter* sp. 806, *Derxia gummosa, Hydrogenophaga flava, Hydrogenobacter thermophilus, Hydrogenophaga palleronii, Hydrophaga pseudoflava, Hydrogenophaga taeniospiralis, Mycobacterium gordonae, Oligotropha carboxidovorans, Paracoccus denitrificans, Pseudomonas carboxydohydrogena, Pseudomonas* sp. 1085, 1749, 2583, 6765, *Pseudomonas saccharophila, Pseudocardia autotrophica, Pseudonocardia petroleophila, Pseudocardia saturnea, Ralstonia eutropha, Variovorax paradoxus, Xanthobacter agilis, Xanthobacter autotrophicus* und *Xanthobacter flavus*. Besonders bevorzugt erfolgt die Markierung in *Ralstonia eutropha* (früher auch als *Alcaligines eutropha* bezeichnet).

Im Gegensatz zu Grünalgen, die zur Fixierung von Kohlendioxid elektromagnetische Strahlung benötigen, nutzen chemolitrotrophe Bakterien chemische Redox-Reaktionen, bei denen anorganische Substrate als Wasserstoffdonatoren genutzt werden, als Energiequelle. Als Kohlenstoffquelle für den Aufbau von Zellsubstanz dient Kohlendioxid, das im Calvin-Zyklus fixiert wird. Die chemolithotrophen Bakterien werden in der Regel in folgende Untergruppen eingeteilt: (i) nitrifizierende Bakterien, in deren Energiestoffwechsel Ammonium zu Nitrit und weiter zu Nitrat bzw. Salpetersäure oxidiert wird, (ii) Schwefelbakterien, die ihre Stoffwechselenergie durch Oxidation einer oder verschiedener reduzierter oder teilweise reduzierter Schwefelverbindungen gewinnen, (iii) Eisen- und Mangan-oxidierende Bakerien, und (iv) Knallgasbakterien, zu denen beispielsweise *Ralstonia eutropha* und die Gattung *Hydrogenomonas* gehören und die die erforderliche Energie aus der Knallgasreaktion gewinnen, deren Wachstum also die Anwesenheit von Wasserstoff und Sauerstoff erfordert. Im Gegensatz zu den meisten Schwefel- und den nitrifizierenden Bakterien sind die sog. Knallgasbakterien nur fakultativ chemoautotroph. Das bedeutet, daß sie in Gegenwart organischer Substanzen wie die meisten Bakterien und die tierischen Organismen heterotroph wachsen und in Angleichung an die herrschenden Wachstumsbedingungen auf den jeweils adäquaten Stoffwechseltyp umschalten. Dies bietet den Vorteil, daß z.B. genetische Manipulationen an *Ralstonia eutropha* und anderen Knallgasbakterien bequem im komplexen Medium ohne H₂ und O₂ durchgeführt werden können.

Die erfindungsgemäße Isotopenmarkierung ist kostengünstiger und weniger aufwendig als die Verfahren des Standes der Technik. Insbesondere zeichnet sich das erfindungsgemäße Verfahren durch den Einsatz weniger aufwendiger und weniger komplexer Wachstumsmedien aus. Auch entfällt im Fall der Markierung mit C-Isotopen, beispielsweise dem stabilen ¹³C-Isotop, der Umweg über die Aufzucht von Grünalgen auf entsprechend markiertem, beispielsweise ¹³C-markiertem, Kohlendioxid und die Herstellung von Algenhydrolysat.

Des weiteren lassen sich CO₂-fixierende Bakterien, wie *Ralstonia eutropha* und andere Stämme, in den Fällen, in denen eine Markierung in *Escherichia coli* unumgänglich ist, für die Produktion von isotopenmarkiertem Hydrolysat verwenden, ähnlich wie Grünalgen und deren Hydrolysat.

Ein weiterer Vorteil des erfindungsgemäßen Verfahrens gegenüber dem Einsatz von Algen liegt darin, daß *Ralstonia eutropha* und ähnliche CO₂-fixierende Stämme zur Fermentation kein Licht benötigen, schneller wachsen als Algen, höhere Zelldichte erreichen, weniger anfällig für Kontaminationen und insgesamt weniger empfindlich als Algen sind, wodurch die Anzucht der Bakterien insgesamt wesentlich leichter und kostengünstiger erfolgen kann.

Ein weiterer Vorteil der *in vivo*-Markierung in chemolithotrophen Bakterien gegenüber der in *E. coli* besteht darin, daß die Konzentration der Kohlenstoffkomponenten während des Wachstums von CO₂-fixierenden Bakterien leichter zu kontrollieren ist, wodurch die homogene Markierung mittels des erfindungsgemäßen Verfahrens leichter durchzuführen ist als mit *E. coli*.

Insgesamt sind die Wachstumsbedingungen beim Einsatz von chemolithotrophen Bakterien im Vergleich zu *E. coli* dank des einfacheren Wachstumsmediums leichter und effizienter kontrollierbar, da die Konzentrationen der Medienbestandteile wesentlich leichter verfolgt werden können.

Weitere Vorteile gegenüber bekannten Verfahren ergeben sich daraus, daß der Nukleinsäure- und Proteinanteil an der Gesamtbiomasse bei *Ralstonia eutropha* und ähnlichen Bakterien höher ist als bei Grünalgen, da Grünalgen einen vergleichsweise höheren Anteil an Kohlenhydraten aufweisen. Hierdurch können u.a. höhere Ausbeuten an isotopenmarkierten Proteinen bzw. Nukleinsäuren erzielt werden.

Des weiteren bietet das erfindungsgemäße Verfahren den Vorteil, daß hierdurch zukünftig auch eine einfache *in vivo*-Markierung von Lipiden möglich ist. Ebenso können mittels des erfindungsgemäßen Verfahrens metabolische Zwischenprodukte und sekundäre Metaboliten aus chemolithotrophen Bakterien, insbesondere CO₂fixierenden Bakterien und besonders bevorzugt aus *Ralstonia eutropha*, in isotopenmarkierter Form hergestellt werden. Durch Verwendung geeigneter Mutanten, deren natürliche metabolische Reaktionswege unterbrochen bzw. gestört sind, können gewünschte Zwischenprodukte angereichert werden. Ein Beispiel für eine geeignete Mutante, die im Rahmen des erfindungsgemäßen Verfahrens geeignet ist, ist die PHB⁻4-Mutante, in der die Produktion von PHB (Polyhydroxybuturat) gestört ist (siehe z.B. Cook and Schlegel (1978) Arch. Microbiol. 119:231-235). Die Mutante PHB⁻4 ist bei der Deutschen Sammlung für Mikroorganismen und Zellkulturen (DSM Braunschweig) z.B. unter der Hinterlegungsnummer DSM 541 hinterlegt. Zwischenprodukte, die beispielsweise in der Mutante PHB⁻4 exkretiert bzw. akkumuliert werden, sind Pyruvat, Malat, Citrat, Fruktose-6-Phosphat und Glukose-6-Phosphat. Prinzipiell ist jede Mutante geeignet, in der wünschenswerte Metabolite exkretiert bzw. angereichert werden. So kann der Metabolismus gezielt oder zufällig gestört sein, so daß durch enzymatischen Ab- oder Aufbau neue Produkte in dem Bakterium entstehen.

In einer besonderen Ausführungsform umfaßt das erfindungsgemäße Verfahren zur *in vivo*-Markierung mit Isotopen die folgenden Schritte:
a) Anzucht der chemolithotrophen Bakterien, insbesondere der Knallgasbakterien, in/auf einem mindestens eine mit einem Isotop markierte Komponente enthaltenden Nährmedium,
b) Ernten der Bakterien und
c) Isolierung des markierten Biopolymers.

In einer bevorzugten Ausführungsform umfaßt die Isolierung des markierten Biopolymers eine LaCl₃-Fällung, wie sie beispielsweise in Abe S. et al. (1987) Agric. Biol. Chem. 51 (6), 1729-1731 beschrieben ist.

Besonders bevorzugt umfaßt die Isolierung von *in vivo*-markierten Proteinen aus chemolithotrophen Bakterien die Schritte:
i) Öffnen der Zellen durch Suspendieren der geernteten Zellen in einem geeigneten Puffer, beispielsweise einem herkömmlichen Tris/EDTA-Puffer, und Zugabe von SDS,
ii) Präzipitation von Nukleinsäuren und Proteinen mittels LaCl₃,
iii) Elution der Proteine durch Suspendieren in SDS-enthaltender Pufferlösung und anschließendes Zentrifugieren,
iv) Präzipitation der Proteine durch beispielsweise Ammoniumsulfat-Fällung.

Die Isolierung von markierten Nukleinsäuren umfaßt bevorzugt die Schritte:
i) Öffnen der Zellen durch Suspendieren der geernteten Zellen in einem geeigneten Puffer, beispielsweise einem herkömmlichen Tris/EDTA-Puffer, und Zugabe von SDS,
ii) Präzipitation von Nukleinsäuren und Proteinen mittels LaCl₃,
iii) Elution der Proteine durch Suspendieren in SDS-enthaltender Pufferlösung und anschließendes Zentrifugieren,
iv) Auflösen des LaCl₃-Pellets in EGTA-enthaltender Pufferlösung (EGTA = Ethylenglycol-bis(beta-aminoethylether)-N,N,N',N'-tetraessigsäure),
v) Phenolextraktion und anschließende Ethanolfällung.

Durch Einsatz des erfindungsgemäßen Verfahrens kann nicht nur eine isotopenreine ¹³C-, ¹¹C- und/oder ¹⁴C-Markierung durch Verwendung von ¹³C- bzw. ¹¹C-, ¹⁴Cmarkiertem Kohlendioxid als Kohlenstoffquelle durchgeführt werden, vielmehr kann die Markierung mit stabilen bzw. instabilen Kohlenstoffisotopen auf einfache Weise durch Wahl eines geeigneten, die entsprechenden Isotope enthaltenen Nährmediums mit der Markierung durch andere Isotope, stabile oder instabile, kombiniert werden. So wie ausschließlich ¹³C- bzw. ¹¹C-, ¹⁴C-markiertes Kohlendioxid als C-Quelle eingesetzt wird, wenn isotopenrein ¹³C- bzw. ¹¹C-, ¹⁴C-markierte Zellmasse von *Ralstonia eutropha* oder anderen chemolithotrophen Bakterien erhalten werden soll, können zusätzlich bspw. ¹⁵N₂ oder ¹⁵N-markierte Salze im Nährmedium enthalten sein, um beispielsweise eine gemischte Markierung mit isotopenreinem ¹³C und ¹⁵N zu erhalten.

Die isotopenreine ¹³C- bzw. ¹¹C-, ¹⁴C-Markierung kann selbstverständlich auch mit einer isotopenreinen Markierung mit Deuterium kombiniert werden. In diesem Fall kann die ²H-Markierung durch Zucht von *Ralstonia eutropha* oder anderen chemolithotrophen Bakterien in D₂O und/oder durch Verfütterung von ²H₂ erfolgen.

Des weiteren kann eine gemischte Markierung mit isotopenreinem ¹³C und ¹⁵N und ²H durchgeführt werden, wobei ein Prozentsatz an ²H von ca. 65-85% bevorzugt wird (vgl. z.B. LeMaster D.M. (1989) Methods in Enzymology 177, 23 ff.). Durch diesen Gehalt an Deuterium wird eine partielle Deuterierung und hierdurch eine Ausdünnen der Spins erreicht.

Die Erfindung betrifft somit in einer besonderen Ausführungsform die *in vivo*-Markierung mit einem C-Isotop in Kombination mit der Markierung mit einem oder mehreren weiteren Isotopen, insbesondere die kombinierte Markierung mit stabilem ¹³C und ¹⁵N und/oder ²H.

Selbstverständlich kann auch eine isotopenreine Markierung mit ¹⁵N durch alleinige Verwendung von ¹⁵N₂ oder ¹⁵N-markierten Salzen im Nährmedium durchgeführt werden. Gleiches gilt für die isotopenreine Markierung mit ausschließlich Deuterium und die gemischte Markierung mit ¹⁵N und ²H. Das erfindungsgemäße Verfahren auf der Basis von Kohlendioxid-fixierenden Bakterien kann grundsätzlich mit allen stabilen oder instabilen Isotopen durchgeführt werden, wobei sich insbesondere stabile oder instabile C-Isotopen in Form von markiertem Kohlendioxid als C-Quelle im Nährmedium anbieten.

Die Erfindung betrifft somit weiter die Verwendung chemolithotropher Bakterien, insbesondere von Knallgasbakterien, zur Markierung von Biopolymeren mit stabilen und/oder instabilen Isotopen, insbesondere mit ¹³C, ¹¹C, ¹⁴C, ¹⁵N, ²H und ⁷⁷Se, einzeln oder in Kombination.

Des weiteren betrifft die Erfindung den Einsatz von gentechnisch veränderten chemolithotrophen Bakterien, bevorzugt Knallgasbakterien, zur Expression *von in vivo*-markierten Proteinen. Hierbei kann die Expression des Proteins, vorzugsweise eines heterologen Proteins, unter Kontrolle eines homologen Promotors oder eines heterologen Promotors (z.B. dem T7-System) stehen. Bevorzugt wird das zu markierende Protein in *Ralstonia eutrophus* unter Kontrolle eines homologen Promotors, beispielsweise des SH (soluble hydrogenase)- oder des MBH (membranebound hydrogenase)-Promotors aus *R. eutrophus*, exprimiert (siehe Schwartz E. *et al*. (1998) Journal of Bacteriology 3197-3204).

Als einen weiteren Aspekt betrifft die vorliegende Erfindung die Verwendung der erfindungsgemäß S-isotopenmarkierten Biomoleküle zur NMR-Spektroskopie.

Hochauflösende NMR-Spektroskopie an großen Molekülen erfordert deren Markierung mit stabilen Isotopen, insbesondere mit den S-Isotopen ²H, ¹³C und ¹⁵N. Eine erfindungsgemäße Verwendung der isotopenmarkierten Biomoleküle ist daher diese sogenannte heteronukleare NMR-Technik

Ebenso betrifft die Erfindung die Verwendung der erfindungsgemäß markierten Biomoleküle bei der bildgebenden NMR-Spektroskopie, bei der die S-isotopenmarkierten Verbindungen als Marker eingesetzt werden, deren Einbau im Organismus verfolgt werden kann.

Weiterhin betrifft die Erfindung den Einsatz von S-isotopenmarkierten Verbindungen in anderen NMR-Techniken, wie beispielsweise der Festkörper-NMR.

Weitere Einsatzmöglichkeiten der erfindungsgemäß markierten Biopolymere bieten sich im Zusammenhang mit der Infrarot- und der Ramanspektroskopie. Durch S-Isotopenmarkierung können die Schwingungsfrequenzen von Molekülgruppen verschoben und somit identifiziert werden. Werden in einem Protein einzelne Aminosäuren oder in einer Nukleinsäure einzelne Nukleotide gezielt S-isotopenmarkiert, können mit diesen spektroskopischen Methoden Aussagen über die Konformation dieser markierten Moleküle erarbeitet werden.

Ein weiteres Einsatzgebiet ist das der Massenspektrometrie. Die Homogenität von Biomolekülen kann über deren Massenverteilung mit Hilfe der Massenspektrometrie analysiert werden. Die Ergebnisse einer solchen massenspektrometrischen Untersuchung sind nur dann eindeutig, wenn die Moleküle homogen bezüglich ihrer Isotopenzusammensetzung sind. Da unsere Umwelt aus einem Gemisch von Isotopen besteht, sind auch die Biomoleküle nicht isotopenrein. So ist zum Beispiel ¹²C in unserer Umwelt mit wenigen Prozenten ¹³C gemischt. Um isotopenreine Biomoleküle herstellen zu können, müssen die Organismen, aus denen die gewünschten Biomoleküle isoliert werden, zuvor auf isotopenreinen Medien angezogen werden.

Des weiteren können S-isotopenmarkierte Biomoleküle bei der Neutronenstreuung eingesetzt werden. Die Isotope ¹H und ²H unterscheiden sich in ihrem Spin voneinander. Da elastische Streueung von Neutronen an den Kemspins erfolgt, können ²H-markierte Proteinkomponenten von unmarkierten Komponenten unterschieden werden. Mit Hilfe der Neutronenstrahlung kann man somit die räumliche Anordnung der markierten Komponenten bestimmen.

Die oben erwähnten Sonden, mit denen S-isotopenmarkierte Verbindungen identifiziert bzw. in einem Organismus verfolgt werden können, haben insgesamt ein großes Anwendungspotential in den Life Sciences, der Medizin, Biologie und Biotechnologie, da sie (i) in den meisten Fällen eine zerstörungsfreie Analyse erlauben und (ii) die hierfür notwendige S-Isotopenmarkierung - mit Ausnahme der Deuterium-Markierung - nicht toxisch ist. Der Einsatz dieser Techniken ist allerdings bislang durch die Verfügbarkeit von S-isotopenmarkierten Biopolymeren begrenzt, da die Markierung mittels herkömmlicher Verfahren kostenintensiv ist und die für die meisten Anwendungen notwendige Homogenität der Markierung mit großem technischen und finanziellen Aufwand verbunden ist.

Durch Einsatz des patentgemäßen Verfahrens werden diese Nachteile überwunden, da die Erfindung eine Markierung zu niedrigeren Kosten und mit höherer Qualität, d.h. insbesondere höherer Homogenität, erlaubt als herkömmliche Verfahren.

Eine übersichtliche Darstellung der genannten Spektroskopie-Techniken, insbesondere der NMR-, Massen-, Infrarot- und Ramanspektroskopiemethoden, bieten Hesse M., Meier, H. und Zeeh B. in "Spektroskopische Methoden in der organischen Chemie", 5. Auflage, 1995, Georg Thieme Verlag Stuttgart.

Des weiteren betrifft die Erfindung die Verwendung von erfindungsgemäß mit instabilen Isotopen, insbesondere ¹¹C oder ¹⁴C, markierten Biomolekülen zur Herstellung eines Medikaments zu diagnostischen und therapeutischen Zwechen, z.B. als Tracerverbindungen.

### Beispiele

### Beispiel 1: Aufzucht von R. eutropha

Stämme von *R. eutropha* können von der Deutschen Sammlung für Mikroorganismen (DSM, Braunschweig) oder der American Type Culture Collection (ATCC) bezogen werden, z.B. der Stamm H 16, der bei der DSM unter der Hinterlegungsnummer DSM 428 und bei der ATCC unter der Nummer 17699 hinterlegt ist.

*R. eutropha* kann in Standardmedien angezogen werden, wie sie beispielsweise in Schwartz *et al*. (1998), supra, oder Eberz G. and Friedrich B. (1991) Journal of Bacteriology 173, 1845-1854 beschrieben sind.

Für chemolithotrophes Wachstum von *R. eutropha* kann beispielsweise das als H-3-Medium bezeichnete Medium eingesetzt werden, das u.a. im "DSMZ-Katalog 1998" (Deutsche Sammlung von Mikroorganismen und Zellkulturen, Mascheroder Weg 1 B, 38124 Braunschweig) beschrieben ist.

| | | |
|---|---|---|
| H-3: | KH₂PO₄ | 2,3 g |
| | Na₂HPO₄ x 2 H₂O | 2,9 g |
| | NH₄Cl | 1,0 g |
| | MgSO₄ x 7 H₂O | 0,5 g |
| | NaHCO₃ | 0,5 g |
| | CaCl₂ x 2 H₂O | 0,01 g |
| | Fe(NH₄)citrat | 0,05 g |
| | Spurenelementelösung SL-6 | 5,0 ml |
| | destilliertes Wasser | 980,0 ml |

| Spurenelementelösung SL-6: | |
|---|---|
| ZnSO₄ x 7 H₂O | 0,1 g |
| MnCl₂ x 4 H₂O | 0,03 g |
| H₃BO₃ | 0,3 g |
| CoCl₂ x 6 H₂O | 0,2 g |
| CuCl₂ x 2 H₂O | 0,01 g |
| NiCl₂ x 6 H₂O | 0,02 g |
| Na₂MoO₄ x 2 H₂O | 0,03 g |
| destilliertes Wasser | 1000 ml |

Nach Einstellen des pH-Werts auf 6,8 wird für 15 min. bei 121 °C autoklaviert. Das Fe(NH₄)citrat (0,05 g in 20 ml H₂O) wird separat sterilisiert und dann zu dem Medium hinzugefügt. Für chemolithotrophes Wachstum der Bakterien wird die Kultur unter einer Atmosphäre von 2 % (v/v) O₂, 10 % CO₂, 60 % H₂ und 28 % N₂ bei einer Temperatur von ca. 80 °C für etwa 4 Stunden inkubiert. Die Atmosphäre wird mit Hilfe von zwei kommunizierenden Gefäßen (je ca. 10 ltr), von denen eines mit Wasser (pH 3-5, um die Löslichkeit von CO₂ im Wasser niedrig zu halten) gefüllt ist. Die verschiedenen Gase werden entsprechend den obigen Angaben in das mit Wasser gefüllte Vorratsgefäß gedrückt. Die Volumina der eingefüllten Gase ergeben sich aus den Volumina des verdrängten Wassers. Das verdrängte Wasser füllt den zweiten 10 ltr Behälter. Die im zweiten Behälter erzeugte Wassersäule dient dazu, das Gasgemisch in den Fermenter zu drücken.

Für heterotrophes Wachstum wird das H-3-Medium durch eine geeignete Kohlenstoffquelle ergänzt (z.B. 0,2 % Kohlenhydrat oder 0,1 % organische Säure). Für Wachstum in/auf Stickstoff-freiem Medium, wird NH₄Cl weggelassen und die Kultur unter einer Atmosphäre von 2 % (v/v) O₂, 10 % CO₂, 10 % H₂ und 78 % N₂ oder heterotroph unter 2% O₂ und 98 % N₂ inkubiert.

Bei der *in vivo*-Markierung werden die entsprechenden Komponenten des Mediums bzw. der Atmosphäre durch entsprechend Isotopen-markierte Verbindungen ersetzt, z.B. wird bei der Markierung mit ¹³C CO₂ durch ¹³CO₂ ersetzt. Bei der Markierung mit ¹⁵N wird entweder N₂ durch ¹⁵N₂ ersetzt oder die N-Salze werden durch ¹⁵N enthaltende Salze ersetzt. Bei der *in vivo*-Isotopenmarkierung mit ²H wird H₂O durch ²H₂O ersetzt und H₂ durch ²H₂.

Eine Markierung mit mehreren Isotopen erfolgt durch Kombination der entsprechenden isotopenmarkierten Komponenten. Partielle Markierung erfolgt durch Änderung der Isotopenkonzentration des entsprechenden Elements.

Markierte Ausgangsstoffe, wie z.B. ¹³C-markiertes CO₂, können beispielsweise von Cambridge Isotope Laboratories (CIL), Massachusetts, USA, bezogen werden.

### Beispiel 2: Isolierung von isotopenmarkierten Proteinen

### a) Öffnen der Zellen

Nach dem Ernten der Bakterien werden 1,0 g Zellen in 10 ml TE-Puffer (100 mM Tris/EDTA pH 7,5) suspendiert, und 1 ml 10% SDS (Sodiumdodecylsulfat) wird hinzugegeben. Anschließend wird die Suspension für 30 min. bei 37 °C und für weitere 30 min. bei 55 °C inkubiert. Die Lösung wird 8mal durch eine Spritze (Durchmesser 0,1 mm) oder bei großen Mengen durch eine Französische Presse (French Press) gedrückt. Dann wird die Lösung für 30 min. bei 18.000 upm zentrifugiert und der erhaltene Überstand in ein Gefäß überführt. Das Bakterienpellet wird erneut in 10 ml TE-Puffer aufgenommen und 1 ml 10% SDS hinzugegeben. Anschließend wird die Suspension wieder für 30 min. bei 37 °C und für weitere 30 min. bei 55 °C inkubiert. Die Lösung wird wiederum durch eine Spritze bzw. eine Französische Presse gedrückt und anschließend für 30 min. bei 18.000 upm abzentrifugiert. Der erhaltene Überstand wird mit dem ersten Überstand vereinigt, so daß das Endvolumen 20 ml beträgt.

### b) Präzipitation von DNA, RNA und Proteinen

20 ml von 20 mM LaCl₃ werden tropfenweise unter Rühren zu dem Überstand aus Schritt a) hinzugefügt, anschließend wird für 20 min. bei 18.000 upm zentrifugiert. Das Pellet wird mit 10 ml H₂O gewaschen, und anschließend wird erneut für 20 min. bei 18.000 upm zentrifuiert.

### c) Elution der Proteine

10 ml TE-Puffer, enthaltend 1% SDS, werden zu dem Pellet hinzugegeben, und das Pellet wird darin gründlich suspendiert. Anschließend wird für 15 min. bei 18.000 upm zentrifugiert und der Überstand in ein frisches Gefäß überführt. Erneut werden 10 ml TE-Puffer, enthaltend 1% SDS, zu dem Pellet hinzugefügt und nach Resuspendierten des Pellets der Zentrifugationsschritt wiederholt. Der die Proteine enthaltende Überstand wird mit dem ersten Überstand vereinigt. Die Proteine können dann beispielsweise durch eine Ammoniumsulfatfällung präzipitiert werden, welche durch Zugabe von 7,0 g (NH₄)₂SO₄ (also 3,5 g (NH₄)₂SO₄ pro 10 ml Lösung) bewirkt werden kann.

### Beispiel 3: Isolierung von isotopenmarkierter DNA und RNA

Das in Schritt b) in Beispiel 2 erhaltene LaCl₃-Pellet wird in 10 ml EGTA-Puffer (0,2 M Trisbase, 50 mM EGTA (Ethylenglycol-bis(b-aminoethylether)-N,N,N',N'tetraessigsäure), 50 mM KOH, 25 mM Mg(OAc)₂) aufgenommen, und 10 ml neutrales Phenol werden hinzugegeben. Im Anschluß an die Deproteinisierung wird das Gemisch zentrifugiert, und DNA und RNA werden aus der wäßrigen Phase mit 2 Volumeneinheiten Ethanol ausgefällt.

Für die Isolierung von RNA wird das DNA/RNA-Pellet zunächst in 10 ml TE-Puffer aufgenommen. Dann werden 300 µl 1 M MgCl₂ (bis 0.03 M) und 1,0 ml 1 M NaAc, pH 4,0, hinzugegeben. Anschließend werden 10 ml Phenol, gesättigt mit 0.1 M NaAc, pH 4,0, hinzugefügt und die Suspension nach gründlichem Mischen zentrifugiert. Die wäßrige Phase wird in ein neues Gefäß überführt, und zu der Phenolphase werden erneut 10 ml TE-Puffer hinzugegeben. Im Anschluß an das Mischen und Zentrifugieren wird die wäßrige Phase abgenommen und mit der ersten wäßrigen Phase vereinigt. Zu der gesammelten wäßrigen Phase (20 ml Volumen) werden 2 ml 2 M Trisbase hinzugegeben und die RNA mit 2,5 bis 3 Volumeneinheiten Ethanol gefällt.

Für die Isolierung von DNA werden zu der Phenolphase 10 ml 10 mM EDTA (Ethylendiamintetraessigsäure) und 1 ml 2 M Trisbase hinzugegeben. Die Suspension wird anschließend sorgfältig gemischt und dann bei 80 °C. für 5 min. erhitzt. Im Anschluß daran wird die Suspension für 5 min. geschüttelt und anschließend zentrifugiert.

Die wäßrige Phase wird abgenommen und in ein frisches Gefäß überführt. Zu der Phenolphase werden eneut 10 ml 10 mM EDTA und 1 ml 2 M Trisbase hinzugegeben. Anschließend wird die Suspension wiederum sorgfältig gemischt und bei 80 °C für 5 min. erwärmt. Nach Schütteln für 5 min. und anschließender Zentrifugation wird die wäßrige Phase mit der ersten wäßrigen Phase vereinigt.

Die DNA wird aus der gesammelten wäßrigen Phase (20 ml Volumen) mit 2 Volumeneinheiten Ethanol und 0,6 ml 3 M NaAc, pH 4,8, ausgefällt.

Die Ausbeuten an DNA, RNA und Protein pro 1 g Zellen von *Ralstonia eutrophus* sind wie folgt:

| | |
|---|---|
| DNA | ca. 10,4 mg |
| RNA | ca. 11.4 mg |
| Protein | ca. 110 mg |

### Beispiel 4: Herstellung eines gentechnisch veränderten Bakteriums für die Expression heterologer Proteine

Das Vektorplasmid pCH 591 (3,1 kb) basierend auf LITMUS 29, New England Biolabs, wurde für die Klonierung von *E. coli* Chaperonin-GroESL-Genen verwendet. Dieses Plasmid trägt einen Ampicillin-Resistenzmarker und ein DNA-Fragment von *Ralstonia eutropha*, welches die Promotorregion sowie die Shine-Dalgarno-Sequenz des lösliche Hydrogenase (soluble hydrogenase, SH)-Gens von *R. eutropha* umfaßt, gefolgt durch einen Polylinker. Plasmid pCH 591 ist in Abbildung 1 veranschaulicht. Als Quelle für die GroESL-Gene diente das Plasmid pOF 39 (R. Stegmann (1999) Dissertation, Technische Universität München). Wie in Abbildung 2 dargestellt, wurde zum einen ein 2,5 kb langes Eco RI/Hind III-Fragment aus pOF 39 isoliert, welches die GroESL-Gene enthält. Das über Agrarosegelelektophorese isolierte Eco RI/Hind III-Fragment wurde anschließend mit dem Restriktionsenzym Bsa AI verdaut, um Blunt-Enden zu erhalten. Diese Behandlung führte zur Eliminierung des *E. coli*-Promotors und eines Teils der Transkriptionssequenz, einschließlich dem +1-Transkriptionsinitiationsnukleotid. Dabei blieb die DNA-Region, enthaltend die Shine-Dalgarno-Sequenz sowie das Initiationskodon für die Proteinsynthese, erhalten. Der Vektor pCH 591 wurde mit dem Restriktionsenzym Nde I behandelt und die Sticky-Enden mit Klenow DNA Polymerase aufgefüllt, gefolgt durch Dephosphorylierung unter Verwendung von alkalischer Phosphatase. Schließlich wurde der behandelte Vektor mit dem Bsa AI-Fragment, enthaltend das GroESL-Gen, unter Einsatz von T4-DNA-Ligase ligiert und die süperkompetenten Zellen Solo Pack™ Gold Cells (Stratagene) mit dem Ligationsansatz transformiert. Das gewünschte Konstrukt enthält zwei Shine-Dalgarno-Sequenzen, nämlich eine aus *E. coli* und eine weitere aus *R. eutropha*. In einer zweiten Klonierungsstrategie zur Klonierung der GroESL-Gene wurde die Shine-Dalgarno-Sequenz von *E. coli* eliminiert, d.h. die gewünschten Konstrukte enthielten nur die Shine-Dalgarno-Sequenz aus *R. eutropha*. Im Rahmen dieser Strategie wurde das Eco RI/Hind III - GroESL-Fragment mit den Restriktionsenzymen Bsa AI und SspI behandelt, wobei letzteres das Fragment an der Stelle der ersten zwei Kodons des GroESL-Gens schneidet, wodurch diese zwei Kodons sowie die *E. coli*-Shine-Dalgarno-Sequenz eliminiert werden. Nach Behandlung des Vektors pCH 591 mit dem Restriktionsenzym Nde I wurde das in Abbildung 2 gezeigte 10-mer Oligonukleotid an die Enden des linearisierten DNA-Fragments ligiert. Durch dieses Oligonukleotid wird nicht nur die Nde I-Schnittstelle wiederhergestellt, das Oligonukleotid enthält eine Nco I-Schnittstelle und kodiert für die Aminosäure Met sowie die zwei Aminosäuren Pro und Trp (siehe Abbildung 2). Das erhaltene DNA-Fragment wurde mit dem Bsa AI/SspI-Fragment, enthaltend die GroESL-Gene, ligiert und das Konstrukt in superkompetente Solopack Gold Cells transformiert. Die Transformanten wurden auf LB-Medium enthaltend 75 µg/ml Ampicillin selektioniert und isolierte Plasmid-DNA hinsichtlich der Insertion von GroESL mittels einer Hind III-Spe I-Restriktionsanalyse im Zusammenhang mit der ersten Klonierungsstrategie und durch Nde I- oder Nco I-Restriktionsverdau in Zusammenhang mit der zweiten Klonierungsstrategie analysiert. Anschließend wurde auch die korrekte Orientierung des GroESL-Fragments durch geeignete Restriktionsverdaue überprüft. Zusätzlich wurden die gewünschten Fusions-übergänge mittels Sequenzierung verifiziert. Im nächsten Schritt wurde das Hind III-Spe I-Fragment, enthaltend den *R. eutropha*-Promotor, Shine-Dalgarno-Sequenz und GroEL/GroES-Gen in einen Wide Host Range Vektor, im vorliegenden Fall in den Vektor pEDY309 (21,2 kb) (abgeleitet aus pEDY305, Schwartz et al. (1998) supra) transferiert und das erhaltene Konstrukt in superkompetente Solo Pack™ Gold Cells transformiert. Das Plasmid pEDY 309 enthält ein Tetracyclin-Resistenzgen, weshalb die Transformanten auf LB-Medium, enthaltend 10 µg/ml Tetracyclin, kultiviert wurden. Die isolierte Plasmid DNA wurde durch Hind III-Restriktionsverdaue analysiert und zusätzlich die gewünschte Orientierung durch Sequenzreaktionen und ergänzend durch PCR-Reaktionen überprüft. Das gewünschte DNA-Konstrukt wurde in *E. coli* S 17-1 retransformiert und anschließend Konjugation von *R. eutropha* H16-Zellen mit den retransformierten *E. coli*-Zellen nach konventionellem Verfahren durchgeführt. Konjuganten wurden in Standardmedium, enthaltend 0,4 % Succinat und 10 µl/ml Tetracyclin selektioniert (siehe auch Schwartz et al. (1998), supra).

### Beispiel 5: Expression heterologer Proteine in R. eutropha am Beispiel des E. coli Chaperonin GroEL/GroES-Proteins

Die rekombinanten *R. eutropha*-Zellen aus Beispiel 4 wurden in Mineral-Medium, enthaltend 0,2 % Fruktose, 0,2 % Glycerol und 10 µg/ml Tetracyclin kultiviert. Nach Wachstum der Zellen wurden die Zellen durch Zentrifugation geerntet, in 100 µl von 10 mM HEPES, pH 7,5, resuspendiert. 100 µl 10% SDS wurden hinzugefügt und nach Erwärmung für 2 Minuten bei 95°C wurden 20 µl 0,1 M MgCl₂ zu der Suspension hinzugefügt. Nach Entfernen des DNA-Pellets durch Zentrifugation wurden 10 µl-Aliquots des Überstandes in der SDS-Gelelektrophorese analysiert. Das Ergebnis der Expression von GroEL in *R. eutropha* ist in dem in Abbildung 3 gezeigten SDS-Gel gezeigt. Die Zellen wurden geöffnet und der Zellinhalt ohne weitere Reinigung auf das Gel aufgetragen. Spur 2 zeigt das Proteinmuster von *R. eutropha* mit dem Expressionsplasmid ohne GroEL/GroES-Gen, Spur 3 - 10 mit GroEL/GroES-Gen. Zwei Klone (9 und 32) sind dargestellt, beide in beiden Orientierungen ("direct": in Uhrzeigerrichtung und "reverse": gegen die Uhrzeigerrichtung) in Gegenwart (+) und ohne (-) Tetracyclin. Spur 1 und 11 zeigt jeweils Referenzproteine, GroEL aus *E. coli* (Spur 1) und Untereinheiten der RNA-Polymerase aus *E. coli* (Spur 10) als Molekulargewichtsstandard. Vergleich der Proteinmuster in den Spuren 2 (Expression ohne *E. coli* GroEL-Gen) mit denen in den Spuren 3 - 10 zeigt, daß eine Bande mit einem Molekulargewicht von 57 kDa fehlt. Diese Band enspricht dem *E. coli* GroEL-Protein. Aus dem Fehlen der Bande in Spur 2 kann geschlossen werden, daß *E. coli* GroEL erfolgreich in *R. eutropha* exprimiert wurde. Dies konnte durch Westernblotting bestätigt werden. Die Expression des heterologen GroEL-Proteins liegt in derselben Größenordnung wie das homologe GroEL.

## Patentansprüche

1. Verfahren zur *in vivo*-Markierung von Biopolymeren mit Isotopen, worin mindestens ein Isotop durch chemolithotrophe Bakterien inkorporiert wird.

2. Verfahren gemäß Anspruch 1, worin das Bakterium ein CO₂fixierendes Bakterium ist.

3. Verfahren gemäß Anspruch 1 oder 2, worin das Bakterium ein Knallgasbakterium ist.

4. Verfahren gemäß einem der vorangehenden Ansprüche, worin das Bakterium *Ralstonia eutropha* ist.

5. Verfahren gemäß einem der vorangehenden Ansprüche, worin das Verfahren die Schritte umfaßt:
a) Aufzucht der Bakterien in/auf einem Medium, das isotopenmarkiertes Kohlendioxid enthält und
b) Ernten der Bakterien und Isolierung der markierten Biopolymere.

6. Verfahren nach einem der vorangehenden Ansprüche, worin die Isolierung der markierten Biopolymere eine LaCl₃-Fällung umfaßt.

7. Verfahren gemäß einem der vorangehenden Ansprüche, worin das Isotop ein S-Isotop ist.

8. Verfahren gemäß einem der vorangehenden Ansprüche, worin das Isotop das Isotop ¹³C ist.

9. Verfahren nach Anspruch 8, worin zusätzlich zu ¹³C mindestens ein weiteres stabiles Isotop durch das Bakterium inkorporiert wird.

10. Verfahren gemäß Anspruch 9, worin das weitere Isotop ¹⁵N und/oder ²H ist.

11. Verfahren gemäß einem der Ansprüche 1 bis 6, worin das Isotop ein instabiles Isotop ist.

12. Verfahren gemäß Anspruch 11, worin das Isotop ¹¹C und/oder ¹⁴C ist.

13. Verfahren gemäß einem der vorangehenden Ansprüche, worin die Biopolymere Zellbestandteile, Nukleinsäuren, Lipide, Kohlenhydrate, Proteine, einschließlich heterolog exprimierte Proteine, metabolische Zwischenprodukte und/oder sekundäre Metaboliten sind.

14. Verwendung eines durch ein Verfahren gemäß einem der vorangehenden Ansprüche erhaltenen isotopenmarkierten Biopolymers zur Herstellung eines Medikamentes zu diagnostischen und/oder therapeutischen Zwecken.

15. Verwendung eines durch ein Verfahren gemäß einem der Ansprüche 1 bis 10, 13 oder 14 erhaltenen mit minestens einem S-Isotop markierten Biopolymers in einem NMR-Verfahren, insbesondere der hochauflösenden NMR-Spektroskopie und der bildgebenden NMR-Spektroskopie, in einem Infrarot- oder Ramanspektroskopieverfahren, der Massenspektrometrie oder einem Neutronenstreuungsverfahren.

16. Verwendung eines durch ein Verfahren gemäß einem der Ansprüche 1 bis 6, 11 oder 12 erhaltenen mit mindestens einem instabilen Isotop markierten Biopolymers als Tracerverbindung bzw. als Marker.

17. Verwendung von chemolithotrophen Bakterien zur Markierung von Biopolymeren mit mindestens einem Isotop.

18. Verwendung gemäß Anspruch 17, worin das Bakterium ein CO₂fixierendes Bakterium, vorzugsweise ein Knallgasbakterium, ist.

19. Verwendung gemäß Anspruch 17 oder 18, worin das Bakterium ein Knallgasbakterium, vorzugsweise *Ralstonia eutropha* ist.

20. Verwendung gemäß einem der Ansprüche 17 bis 19, worin das mindestens eine Isotop ein stabiles Isotop ist.

21. Verwendung gemäß Anspruch 20, worin das stabile Isotop ¹³C, ¹⁵N und/oder ²H ist.

22. Verwendung gemäß einem der Ansprüche 17 bis 19, worin das mindestens eine Isotop ein instabiles Isotop ist.

23. Verwendung gemäß Anspruch 22, worin das instabile Isotop ¹¹C oder ¹⁴C ist.

## Claims

1. A method for *in vivo* labeling of biopolymers with isotopes, wherein at least one isotope is incorporated by chemolithotrophic bacteria.

2. The method according to claim 1, wherein the bacterium is a CO₂-fixing bacterium.

3. The method according to claim 1 or 2, wherein the bacterium is a hydrogen bacterium.

4. The method according to any of the preceding claims, wherein the bacterium is *Ralstonia eutropha*.

5. The method according to any of the preceding claims, wherein the method comprises the steps:
a) cultivating the bacteria in/on a medium, which contains isotope labeled carbon dioxide and
b) harvesting the bacteria and isolating the labeled biopolymers.

6. The method according to any of the preceding claims, wherein the isolation of the labeled biopolymers comprises a LaCl₃ precipitation.

7. The method according to any of the preceding claims, wherein the isotope is an S isotope.

8. The method according to any of the preceding claims, wherein the isotope is the ¹³C isotope.

9. The method according to claim 8, wherein in addition to ¹³C at least one further stable isotope is incorporated by the bacterium.

10. The method according to claim 9, wherein the additional isotope is ¹⁵N and/or ²H.

11. The method according to any of the claims 1 to 6, wherein the isotope is an instable isotope.

12. The method according to claim 11, wherein the isotope is ¹¹C and/or ¹⁴C.

13. The method according to any of the preceding claims, wherein the biopolymers are cell components, nucleic acids, lipids, carbohydrates, proteins including heterologously expressed proteins, metabolic intermediate products and/or secondary metabolites.

14. Use of an isotope-labeled biopolymer obtained by a method according to any of the preceding claims for the production of a medicament for diagnostic and/or therapeutic purposes.

15. Use of a biopolymer labeled with at least one S isotope and obtained by a method according to any of the claims 1 to 10, 13 or 14 in an NMR method, especially the high-resolution NMR spectroscopy and imaging NMR spectroscopy, in an infrared or Raman spectroscopy method, mass spectrometry or a neutron scattering technique.

16. Use of a biopolymer labeled with at least one instable isotope and obtained by a method according to any of the claims 1 to 6, 11 or 12 as a tracer compound or as a label, respectively.

17. Use of chemolithotrophic bacteria for labeling of biopolymers with at least one isotope.

18. Use according to claim 17, wherein the bacterium is a CO₂-fixing bacterium, preferably a hydrogen bacterium.

19. Use according to claim 17 or 18, wherein the bacterium is a hydrogen bacterium, preferably *Ralstonia eutropha*.

20. Use according to any of the claims 17 to 19, wherein the at least one isotope is a stable isotope.

21. Use according to claim 20, wherein the stable isotope is ¹³C, ¹⁵N and/or ²H.

22. Use according to any of the claims 17 to 19, wherein the at least one isotope is an instable isotope.

23. Use according to claim 22, wherein the instable isotope is ¹¹C or ¹⁴C.

## Revendications

1. Procédé de marquage isotopique de biopolymères *in vivo*, dans lequel au moins un isotope est incorporé par des bactéries chimiolithotrophes.

2. Procédé selon la revendication 1, dans lequel la bactérie est une bactérie fixant le CO₂.

3. Procédé selon la revendication 1 ou la revendication 2, dans lequel la bactérie est une bactérie oxydant l'hydrogène (bactérie Knallgas).

4. Procédé selon l'une des revendications précédentes, dans lequel la bactérie est *Ralstonia eutropha*.

5. Procédé selon l'une des revendications précédentes, dans lequel le procédé comprend les étapes suivantes :
a) culture des bactéries dans/sur un milieu de culture contenant du dioxyde de carbone marqué avec un isotope, et
b) récolte des bactéries et isolation des biopolymères marqués.

6. Procédé selon l'une des revendications précédentes, dans lequel l'isolation des biopolymères marqués comprend une précipitation au LaCl₃.

7. Procédé selon l'une des revendications précédentes, dans lequel l'isotope est un isotope de S.

8. Procédé selon l'une des revendications précédentes, dans lequel l'isotope est l'isotope ¹³C.

9. Procédé selon la revendication 8, dans lequel, en plus du ¹³C, au moins un autre isotope stable est incorporé par la bactérie.

10. Procédé selon la revendication 9, dans lequel l'autre isotope est le ¹⁵N et/ou le ²H.

11. Procédé selon l'une des revendications 1 à 6, dans lequel l'isotope est un isotope instable.

12. Procédé selon la revendication 11, dans lequel l'isotope est le ¹¹C et/ou le ¹⁴C.

13. Procédé selon l'une des revendications précédentes, dans lequel les biopolymères sont des composants cellulaires, des acides nucléiques, des lipides, des hydrates de carbone, des protéines, y compris des protéines exprimées de manière hétérologue, des produits intermédiaires du métabolisme et/ou des métabolites secondaires.

14. Utilisation d'un biopolymère marqué avec un isotope, obtenu par un procédé selon l'une des revendications précédentes, pour préparer un médicament à usage diagnostique et/ou thérapeutique.

15. Utilisation d'un biopolymère marqué avec au moins un isotope de S, obtenu par un procédé selon l'une des revendications 1 à 10, 13 ou 14, dans un procédé de RMN, en particulier la spectroscopie par RMN à haute résolution et la spectroscopie à imagerie par RMN, dans un procédé de spectroscopie infrarouge ou Raman, dans un procédé de spectrométrie de masse ou dans un procédé à diffusion de neutrons.

16. Utilisation d'un biopolymère marqué avec au moins un isotope instable, obtenu par un procédé selon l'une des revendications 1 à 6, 11 ou 12, comme composé traceur ou comme marqueur.

17. Utilisation de bactéries chimiolithotrophes pour le marquage de biopolymères avec au moins un isotope.

18. Utilisation selon la revendication 17, dans laquelle la bactérie est une bactérie fixant le CO₂, de préférence une bactérie oxydant l'hydrogène (bactérie Knallgas).

19. Utilisation selon la revendication 17 ou la revendication 18, dans laquelle la bactérie est une bactérie oxydant l'hydrogène (bactérie Knallgas), de préférence *Ralstonia eutropha*.

20. Utilisation selon l'une des revendications 17 à 19, dans laquelle le ou les isotopes sont stables.

21. Utilisation selon la revendication 20, dans laquelle l'isotope stable est le ¹³C, le ¹⁵N et/ou le ²H.

22. Utilisation selon l'une des revendications 17 à 19, dans laquelle le ou les isotopes sont instables.

23. Utilisation selon la revendication 22, dans laquelle l'isotope instable est le ¹¹C ou le ¹⁴C.
